# EUROPÄISCHE PATENTSCHRIFT

(11) **EP 2 514 469 B1**
(45) Veröffentlichungstag und Bekanntmachung des Hinweises auf die Patenterteilung: **11.01.2017**
(21) Anmeldenummer: 12002675.2
(22) Anmeldetag: 17.04.2012
(51) Int. Cl.: A61M 16/00

(54) **Vorrichtung zur Absenkung des Einatmungsdruckniveaus**
Device for reducing ventilation pressure
Dispositif d'abaissement du niveau de pression d'inspiration

(30) Priorität: 19.04.2011 DE 102011018220
(43) Veröffentlichungstag der Anmeldung: 24.10.2012
(73) Patentinhaber: Löwenstein Medical Technology S.A., 2557 Luxembourg (LU)
(72) Erfinder: Göbel, Christof, 22457 Hamburg (DE); Feldhahn, Karl-Andreas, 22761 Hamburg (DE)
(74) Vertreter: Marx, Thomas

(56) Entgegenhaltungen:
- EP-A2- 0 943 353
- WO-A1-99/45989
- US-A- 3 961 627
- US-A1- 2006 011 195

## Beschreibung

Die Erfindung bezieht sich auf eine Vorrichtung, welche den Einatmungsdruck gezielt absenkt und so die Zeitdauer und den Verlauf der Ausatemphase steuert.

Derartige Vorrichtungen können insbesondere im Zusammenhang mit Beatmungsgeräten zum Einsatz kommen, die für Patienten mit COPD-Erkrankungen verwendet werden. Typische Krankheitsbilder sind Emphysem und chronisch obstruktive Bronchitis. Diese Erkrankungen führen dazu, dass es im Rahmen einer Beatmung in der Exspiration zu einem Kollaps kleiner Atemwege kommen kann. Entsprechend ist die Exspiration unvollständig und ein erhöhter intrapulmonaler Druck besteht. Die Behinderung der Ausatmung führt zu einem intrathorakalen Druckanstieg. Daraus folgt eine Minderbelüftung der Lunge.

Die EP 0 943 353 A2 offenbart, dass durch eine Parametervorgabe eine Beeinflussung der Beatmungsdruckkurven in der Einatmungsphase erfolgt, um Spitzendruckverläufe im Bereich der Atemwege des Patienten zu reduzieren. Dazu werden unterschiedliche Druckbegrenzungen vorgegeben. Die Steuereinrichtung für das Absenken des Druckes weist eine regelbare Atemgasquelle auf, um einem IPAP-Druck bereitzustellen, Sensor-Mittel, um Druck- und/oder Fluss-Werte des Atemgases zu ermitteln, Prozessor-Mittel zum Verarbeiten der vom Sensor ermittelten Werte, Speicher-Mittel zum abrufbaren Vorhalten von Funktionen, Eingabemittel um die Funktionen zumindest teilweise zu bestimmen und Steuermittel welche die Atemgasquelle, entsprechend der Funktionen steuern.

Die US 2006/011195 A1 offenbart, dass zunächst ein Basisdruck vorgegeben wird, welcher dann auf ein Ausatemniveau abgesenkt und anschließend auf einen Spitzenwert angehoben wird. Daraufhin wird der Druck wieder auf den Basisdruck abgesenkt. Der Spitzenwert liegt dabei immer über dem Basisdruck liegt. Die US 2006/011195 A1 offenbart drei unterschiedliche Druckniveaus für die Beatmung. Die Druckabsenkungen erfolgen hier passiv.

Die WO 99/45989 offenbart eine Ausatemerleichterung, bei der der exspiratorische Basisdruck (EPAP) noch unterschritten wird. Hierbei wird abhängig von dem Atemgasfluss ein Verstärkungsfaktor ermittelt und die Ausatemerleichterung wird mit dem Verstärkungsfaktor multipliziert.

Die US 3,961,627 offenbart die Automatisierung einer druckkontrollierten oder volumenkontrollierten Beatmung. Hierbei werden vier Phasen unterschieden, wobei die Phasen III und IV der Exspiration dienen. Die Exspirationsphasen sind druckkontrolliert, wobei der Arzt den Druckabfall vorgeben kann. Die US 3,961,627 offenbart zudem eine Beeinflussung des exspiratorischen Druckverlaufes in zwei Phasen, wobei die die Zeitdauer der Phase IV nie kürzer ist als die Dauer der Phase III ist, um eine Überblähung der Lunge (air trapping) zu vermeiden.

Aufgabe der vorliegenden Erfindung ist es, eine Vorrichtung anzugeben, die durch eine intelligente und störungstolerante Gerätesteuerung eine ausreichende Exspiration unterstützen. Idealerweise dergestalt, dass die Atemwege während der Exspiration, durch einen ausreichenden Gegendruck, dauerhaft geöffnet bleiben.

Diese Aufgabe wird erfindungsgemäß durch die Merkmale des Anspruchs 1 gelöst.

Die vorliegende Erfindung betrifft daher die schrittweise Absenkung des inspiratorischen IPAP-Druckes (inspiratory positive airway pressure) auf ein endexspiratorisches Niveau. Das PEEP Niveau ist bevorzugt größer Null, kann aber auch zu Null (ZEEP = Zero end exspiratory pressure) gewählt werden. In der ersten Phase wird durch eine erste Funktion ein schneller Druckabfall bewirkt, zur Unterstützung einer effizienten Ausatmung. Bevorzugt ist die erste Funktion eine steile Rampe mit vorbestimmter erster Charakteristik. Eine Zeitdauer im Bereich 1% bis 10% der Ausatemzeit (Te) wurde als günstiger Wert für die Dauer der ersten Phase I ermittelt. Typischerweise wird der IPAP-Druck in der ersten Phase I um zumindest 25%, bevorzugt um zumindest 35% abgesenkt. Mit Erreichen eines Zwischenniveaus zu Beginn einer Phase II, steuert eine zweite Funktion einen - im Vergleich zur ersten Funktion - langsameren Druckabfall zur Abbremsung der Ausatmung und zur Verhinderung eines Atemwegskollaps. Dies dient dazu die Atemwege in einer kritischen Phase der Ausatmung zu schienen. Die zweite Charakteristik ist vorwiegend dergestalt, dass das Druckniveau im Wesentlichen gehalten wird oder einen langsamen Druckabfall aufweist. Die zweite Funktion wird dazu für eine Zeitdauer von typisch 15% bis 35% der Ausatemzeit ausgeführt. Bevorzugt ist der Beginn der Anwendung der zweiten Funktion vom Anwender einstellbar. Die Dauer kann als Absolutzeit oder als Anteil der Exspirationszeit vorgegeben werden. Nach Beendigung der zweiten Funktion kann eine dritte Funktion angewendet werden, welche einen schnelleren Druckabfall steuert als die zweite Funktion, wobei die Charakteristik der dritten Funktion von der Charakteristik der ersten Funktion abweichen kann, in einer Ausführungsform aber mit ihr identisch ist. Die dritte Funktion wird dazu für eine Zeitdauer von etwa 5% bis 15% der Ausatemzeit ausgeführt.

Über die erste und dritte Funktion wird ein vergrößerter Fluss realisiert, welcher die Exspiration wirksam unterstützt. Durch die zweite Funktion wird ein Zwischendruckniveau zumindest vorübergehend im Wesentlichen aufrecht erhalten; dadurch wird einem Kollaps von Atemwegen und einem damit verbundenen typischen Einbrechen des Ausatemflows entgegen gewirkt.

Die erfindungsgemäße Vorrichtung zur Steuerung der Ausatemphase ermöglicht vorgebbare Fluss-Verläufe und/oder Druck-Verläufe und/oder Volumen-Verläufe durch eine Steuereinheit zur Vorgabe eines Ausatemmusters und eine mit der Steuereinheit verbundene Messeinrichtung zur Erfassung des Exspirationsverlaufs während einer Ausatemphase hinsichtlich Druck und/oder Fluss und/oder Volumen, sowie über mit der Steuereinheit verbundene Mittel zur Drosselung und/oder Beschleunigung der Ausatmung hinsichtlich Druck und/oder Fluss und/oder Volumen. Bevorzugt können Fluss-Verläufe und/oder Druck-Verläufe und/oder Volumen-Verläufe für die Ausatmung, sowie die Dauer der entsprechenden Verläufe vom Arzt vorgegeben werden. Die erfindungsgemäße Steuerung kann dabei unter der sogenannten mandatorischen/ kontrollierten Beatmung (T, PCV -Modus) angewendet werden oder unter einer assistierten Beatmung (S, ST, PSV - Modus). Im T/PCV- Modus wird die Exspirationszeit Te festgeglegt und die vorgebbaren Fluss- und/oder Druck- und/oder Volumenverläufe werden für die gesamte Zeit Te gesteuert. Im ST/PSV- Modus kann der Patient, gegenüber der eingestellten (maximalen) Ausatemzeit Te, früher eine Einatmung beginnen. In diesem Fall wird der exspiratorische Fluss- und/oder Druck- und/oder Volumenverlauf nur bis zu dem Zeitpunkt im erfindungsgemäßen Sinne gesteuert, bis der Patient mit der Einatmung beginnt.

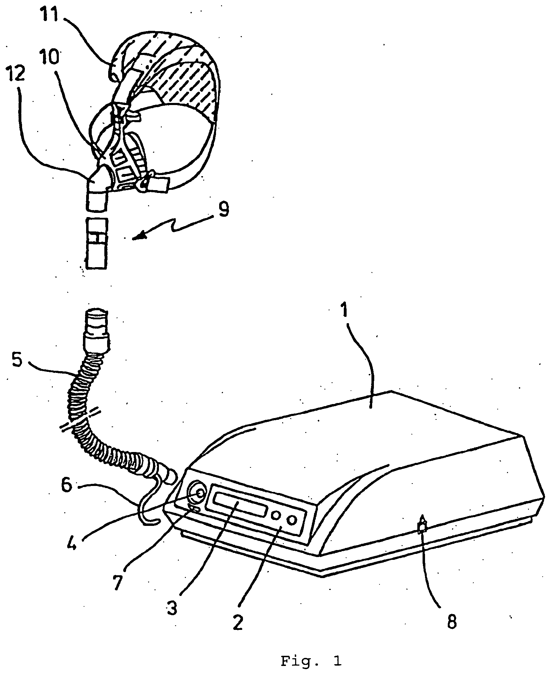

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf. Im Bereich einer dem Gerätegehäuse (1) abgewandten Ausdehnung des Verbindungsschlauches (5) ist ein Ausatmungselement (9) angeordnet. Fig. 1 zeigt darüber hinaus ein Patienten-Interface (10), das als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) einen Anschlussstutzen (12) mit Kugelgelenk auf.

Die Vorrichtung zur Beatmung weist eine Steuereinrichtung für das Absenken eines Einatmungsdruckes (IPAP) auf ein EPAP oder PEEP-Niveau auf die Folgendes umfasst;
- eine regelbare Atemgasquelle, hier als elektronisch betriebener Lüfter ausgeführt, um einem IPAP-Druck bereitzustellen
- Sensor-Mittel, um Druck- und/oder Fluss-Werte des Atemgases zu ermitteln
- Prozessor-Mittel zum Verarbeiten der vom Sensor ermittelten Werte
- Speicher-Mittel zum abrufbaren Vorhalten von Funktionen, die bevorzugt mathematische Funktionen sind, welche - wenn ausgeführt - zu einem Druckabfall mit definierter Charakteristik führen
- Eingabemittel um die Funktionen zumindest teilweise zu bestimmen; wobei darunter die Auswahl hinterlegter Funktionen ebenso verstanden wird, wie die, zumindest teilweise, Eingabe von Funktionen, auch über eine Schnittstelle, und unter "bestimmen" auch das parametrisieren einer hinterlegten Funktion verstanden wird, und hierbei beispielsweise die Zeitdauer einer Phase oder der Druckabfall in einer Phase verändert wird
- Steuermittel welche die Atemgasquelle, entsprechend der Funktionen steuern wobei die Steuermittel ausgehend vom IPAP-Druck in einer ersten Phase die Atemgasquelle zu einem Druckabfall einer ersten Charakteristik (14) (entsprechend einer ersten Funktion) veranlassen, mit einem Druckabfall im Bereich 30
- 300 hPa/sec, bevorzugt 50 - 250 hPa/s, besonders bevorzugt 50 - 200 hPa/s, und dann in einer zweiten Phase die Atemgasquelle zu einem zweiten Druckabfall (15, 16) (entsprechend einer zweiten Funktion) mit einer von der ersten Phase abweichenden Charakteristik veranlassen, wobei der zweite Druckabfall wenigstens abschnittsweise im Bereich kleiner 30 hPa/sec, bevorzugt kleiner oder gleich 25 hPa/sec, besonders bevorzugt kleiner 20 hPa/sec liegt.

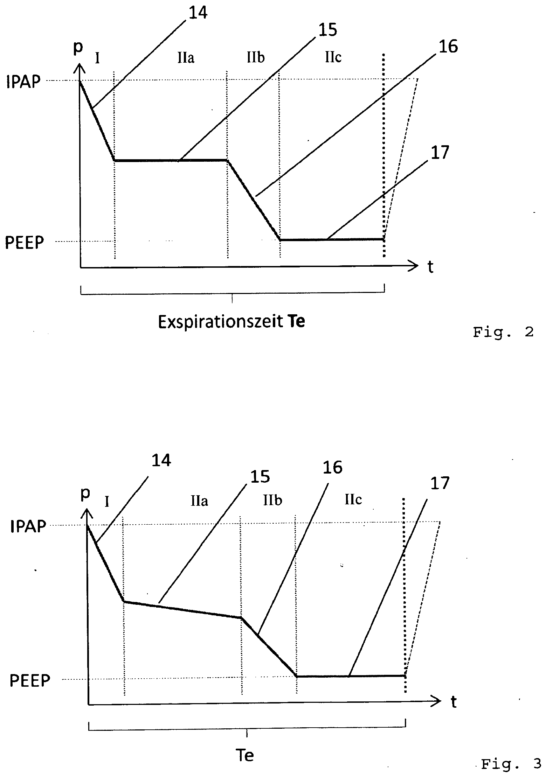

Bei den folgenden Ausführungsformen der Figuren 2 bis 10 wird der inspiratorische IPAP-Druck (inspiratory positive airway pressure) (13) in zumindest zwei Phasen auf ein endexspiratorisches DruckNiveau (PEEP), welches bevorzugt größer Null ist, abgesenkt.

Figur 2 zeigt die schrittweise Absenkung des inspiratorischen IPAP-Druckes (inspiratory positive airway pressure) auf ein endexspiratorisches Niveau. Das PEEP Niveau ist bevorzugt größer Null, kann aber auch zu Null (ZEEP = Zero end exspiratory pressure) gewählt werden. In der ersten Phase I wird durch eine erste Funktion (14) ein schneller Druckabfall bewirkt, zur Unterstützung einer effizienten Ausatmung. Bevorzugt ist die erste Funktion eine steile Rampe mit vorbestimmter erster Charakteristik (14). Eine Zeitdauer im Bereich 1% bis 10% der Ausatemzeit (Te) wurde als günstiger Wert für die Dauer der ersten Phase I ermittelt. Typischerweise wird der IPAP-Druck in der ersten Phase I um zumindest 25%, bevorzugt um zumindest 30%, besonders bevorzugt um zumindest 40% abgesenkt. Mit Erreichen eines Zwischenniveaus zu Beginn einer Phase II, steuert eine zweite Funktion (15) (hier in Phase IIa) einen - im Vergleich zur ersten Funktion - langsameren Druckabfall (=zweite Charakteristik (15)), zur Abbremsung der Ausatmung und zur Verhinderung eines Atemwegskollaps. Dies dient dazu die Atemwege in einer kritischen Phase der Ausatmung zu schienen. Die zweite Charakteristik (15) ist vorwiegend dergestalt, dass das Druckniveau im Wesentlichen gehalten wird oder einen langsamen Druckabfall aufweist. Die zweite Funktion (in Phase IIa) wird dazu für eine Zeitdauer von typisch 15% bis 35% der Ausatemzeit ausgeführt. Bevorzugt ist der Beginn der Anwendung der zweiten Funktion vom Anwender einstellbar. Die Dauer kann als Absolutzeit oder als Anteil der Exspirationszeit vorgegeben werden. Nach Beendigung der zweiten Funktion wird eine dritte Funktion (16) (in Phase IIb) angewendet welche einen schnelleren Druckabfall steuert als die zweite Funktion, wobei die Charakteristik der dritten Funktion (16) von der Charakteristik der ersten Funktion (14) abweichen kann, in einer Ausführungsform aber mit ihr identisch ist. Die dritte Funktion wird dazu für eine Zeitdauer von etwa 5% bis 15% der Ausatemzeit ausgeführt.

Über die erste und dritte Funktion wird ein vergrößerter Fluss realisiert welcher die Exspiration wirksam unterstützt. Durch die zweite Funktion wird ein Zwischendruckniveau vorübergehend und bei verlangsamter Abfallgeschwindigkeit im Wesentlichen aufrecht erhalten; dadurch wird einem Kollaps von Atemwegen und einem damit verbundenen typischen Einbrechen des Ausatemflows entgegen gewirkt. Zum Ende der Phase II ist das PEEP-Niveau (17) erreicht und wird für eine vorbestimmbare Zeit gehalten, bevor die Steuerung den Druck auf das IPAP-Niveau hochregelt. Die Druckabfallgeschwindigkeiten sind bevorzugt in Phase I: 50 - 200 hPa/s, in Phase IIa: 0 - 2 hPa/s, in Phase IIb: 10 - 200 hPa/s.

Figur 3 zeigt in der Phase IIa eine zweite Funktion (15) - anders als in Abb.2 - mit einem definierten Druckabfall (=zweite Charakteristik (15)). Auch in diesem Beispiel wird in der Phase II die Abbremsung der Ausatmung zur Verhinderung eines Atemwegskollaps gesteuert, wobei das Druckniveau in der Phase IIa langsam abgesenkt wird. Die Druckabfallgeschwindigkeiten sind in Phase I: 50 - 200 hPa/s, in Phase IIa: 0 - 20 hPa/s, in Phase IIb: 10 - 200 hPa/s.

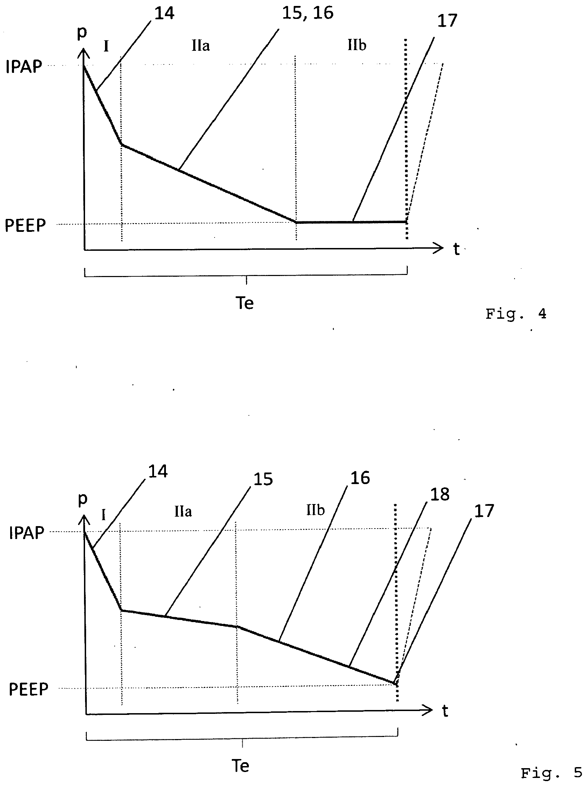

Figur 4 zeigt in der Phase IIa eine einzelne Charakteristik des Druckabfalls bis auf das PEEP Nivau. Auf ein Zwischenniveau der Phase II in der Art der Ausführungsformen aus Figur 2 bzw. Figur 3 wurde hier verzichtet. In diesem Beispiel wird nach einem ersten steilen Druckabfall in der Phase I, die Abbremsung der Ausatmung durch einen zweiten weniger steilen Druckabfall unterstützt, der in den EPAP/PEEP mündet. Die Druckabfallgeschwindigkeiten sind in Phase I: 50 - 200 hPa/s, in Phase IIa: 2 - 20 hPa/s.

Figur 5 zeigt in der Phase IIa eine Charakteristik mit stetigem Druckabfall und eine anschließende Phase IIb mit einem gegenüber der Phase IIa steileren Druckabfall bis auf das PEEP-Niveau. In diesem Beispiel wird der Druck in Phase IIb so langsam abgesenkt, dass der PEEP erst zum Ende der vorgesehenen Exspirationszeit und unmittelbar vor einem Umschaltvorgang auf den Inspirationsdruck erreicht wird. Somit entfällt hier eine ausgeprägte Phase, in der der Druck gleich dem PEEP-Niveau ist. Die Druckabfallgeschwindigkeiten sind in Phase I: 50 - 200 hPa/s, in Phase IIa: 0 - 20 hPa/s, in Phase IIb: 2 - 20 hPa/s.

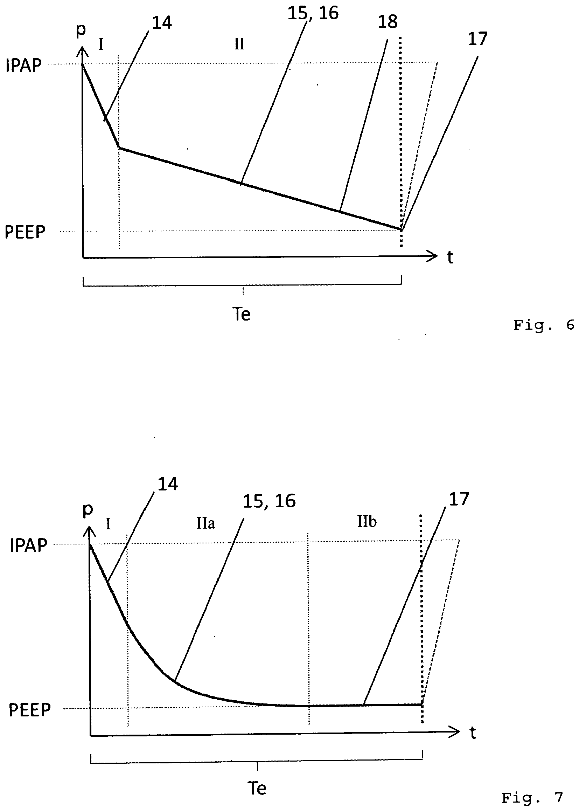

Figur 6 zeigt in der Phase II eine Charakteristik mit stetigem - gegenüber der Phase I verlangsamten - Druckabfall bis auf das PEEP -Niveau. Der Druck wird in dieser Phase so langsam abgesenkt, dass der PEEP erst zum Ende der vorgesehenen Exspirationszeit und unmittelbar vor dem Umschaltvorgang auf den Inspirationsdruck erreicht wird. Somit entfällt hier eine ausgeprägte Phase, in der der Druck gleich dem PEEP-Niveau ist. Die Druckabfallgeschwindigkeiten sind in Phase I: 50 - 200 hPa/s, in Phase II: 2 - 20 hPa/s.

Anhand der Ausführungsformen der Figuren 5 und 6 ist ersichtlich, dass die hier gewählte Charakteristik der Phase II, insbesondere bei einer S- oder ST-Beatmung, geeignet ist, einen Patiententrigger für die nächste Einatmung- bei gegebenem intrinsischen PEEP (PEEPi) wirkungsvoll zu unterstützen. Eine Einatembemühung des Patienten (18) zu einem Zeitpunkt, an dem das PEEP-Niveau noch nicht vollständig erreicht ist - also ein Druck noch über dem PEEP vorliegt - wird wegen des erhöhten Druckniveaus sensorisch gut erkannt, bei verminderter Patientenanstrengung, und gerätetechnisch schnell in einen Druckanstieg auf das IPAP-Niveau umgesetzt.

Figur 7 zeigt ein Beispiel mit einer linearen Anfangssteigung und eine Charakteristik in der Phase IIa mit einem Druckabfall bei sich stetig ändernder Steigung bis auf das PEEP-Niveau, welches vor dem Ablauf der Exspirationszeit erreicht wird. Der schnelle Druckabfall der Phase I wird auf diese Weise in der Phase IIa allmählich verlangsamt. Der Druck geht asymptotisch in den PEEP über und wird für die restliche Exspirationszeit (Te) auf diesem Niveau gehalten (Phase IIb).

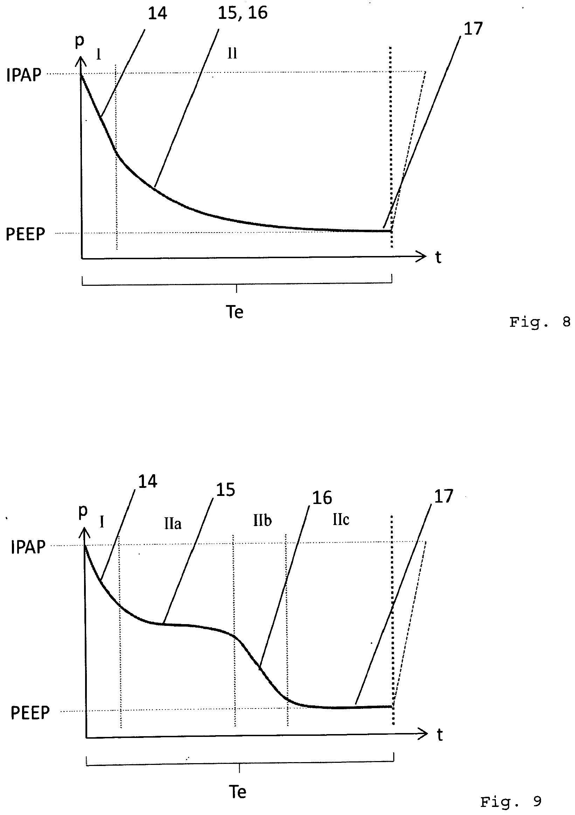

Figur 8 zeigt eine Charakteristik in der Phase II mit einem Druckabfall bei sich stetig ändernder Steigung, bei der das PEEP-Niveau erst zum Ende der Exspiration erreicht wird. In diesem Beispiel ist die Anfangssteigung in der Phase I zunächst konstant, ändert sich aber in der Phase II stetig, wodurch ein schneller Druckabfall in der Phase I allmählich verlangsamt wird. Der Druck geht zum Ende der vorgesehenen Exspirationszeit asymptotisch in den PEEP über, bevor für die nachfolgende Einatmung auf den Inspirationsdruck umgeschaltet wird. In dieser Ausführungsform wird der PEEP erst zum Ende der Ausatemzeit erreicht.

Der Druckübergang der Fig. 9 verdeutlicht die Möglichkeit, in den Übergangsbereichen der Phasen mit weichen Übergängen anstelle von diskontinuierlichen Änderungen des Druckabfalls zu arbeiten. Dieses Beispiel ist übertragbar auf alle Beispiele mit diskontinuierlichen Übergangsstellen und ermöglicht jeweils einen erhöhten Komfort für empfindliche Patienten.

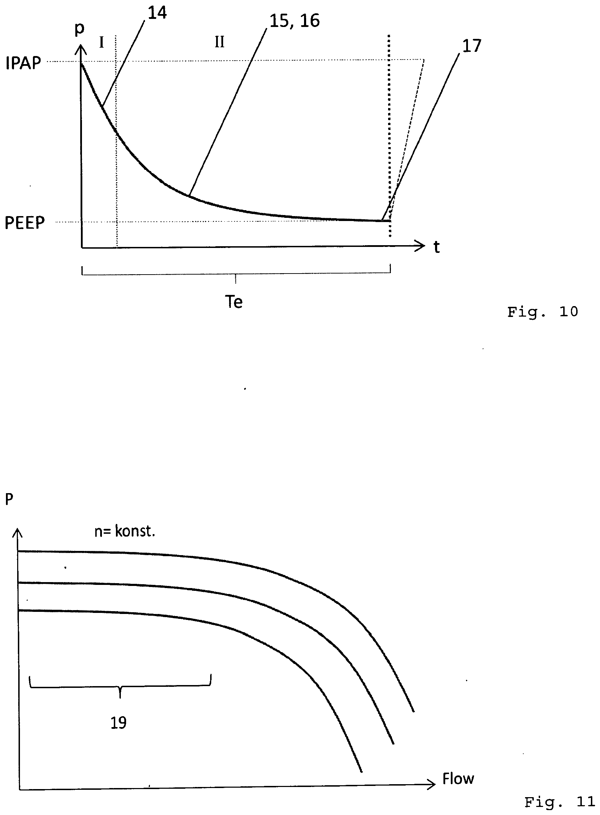

Fig. 10 zeigt einen stetigen Druckübergang in der gesamten vorgesehenen Ausatemzeit Te mit einer großen Anfangssteilheit in der Phase I und einer kontinuierlich abnehmenden Steilheit mit einem asymptotischen Übergang auf den PEEP zum Ende der Phase II.

Bei den vorgenannten Ausführungsformen ist für die erste Phase I eine Zeitdauer im Bereich 1% bis 15%, bevorzugt 5 % bis 10% der Ausatemzeit (Te) vorgesehen. Typischerweise wird der IPAP-Druck in der ersten Phase I um zumindest 25%, bevorzugt um zumindest 35% abgesenkt. Die für restliche Ausatemzeit wird Phase II angewendet, mit den für jede Ausführungsform charakteristischen Funktionen.

In zumindest einer Ausführungsform sind der Druck bzw. der Druckbereich der zweiten Funktion und die Dauer der zweiten Funktion einstellbar und/oder ist auch die Charakteristik der dritten Funktion (16) einstellbar. Sobald der vorgegebene PEEP erreicht wird, wird die Anwendung der dritten Funktion beendet. Für die verbleibende Ausatemzeit findet die Exspiration mit dem eingestellten PEEP statt, wodurch die Exspiration nach einer Phase mit hoher Kollapsneigung der Atemwege wieder durch niedrigen oder minimalen (Zero Peep, ZEEP) Gegendruck optimal unterstützt wird.

Die erfindungsgemäße Vorrichtung muss verschiedene Anforderungen an die Dynamik der verwendeten Atemgasquelle (21) erfüllen. Diese Druckquelle ist vorzugsweise ein Gebläse, dessen Motor mit einem geeigneten Drehmoment für die erforderlichen Bremsbeschleunigungen und eine Motor-Lüftereinheit (21) mit einem möglichst geringen Massenträgheitsmoment aufweist. Die (Brems-) Beschleunigungen lassen sich beispielsweise über Kurzschlussschaltungen der Motorwicklungen oder über ein gegenüber der jeweiligen Rotordrehposition nachlaufendes elektrisches Feld erzielen. Motordrehmoment und Wirkungsgrad sind auf die Eigenschaften des Laufrades abgestimmt.
Das Lüfterrad ist vorzugsweise ein Lüfter mit im Wesentlichen radialer Durchströmung und radialer Beschaufelung, wodurch eine entsprechende Druckkonstanz (19) in den Kennlinien (Drehzahl, Druck, Fluss- Kennlinien) erzielt wird (siehe Figur 11). Die Schaufeln enden zum Erzielen dieser Kennlinien im Wesentlichen radial, in einem Winkel von 90° gegenüber der Umfangsgeschwindigkeit, oder zumindest in einem relativ steilen Winkel (>70°). Dadurch ist in einem gewissen Bereich der Kennlinien (vgl. Abb. 11) der Druck jeweils weitgehend unabhängig vom abgegebenen Fluss und die erfindungsgemäßen Druckverläufe sind real leicht zu erzielen und - gegenüber anderen Bauformen - unabhängiger von Störeinflüssen.

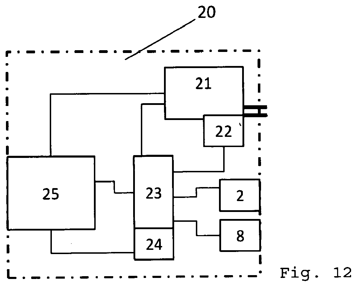

Figur 12 zeigt schematisch die Steuereinrichtung (20) für das Absenken des Einatmungsdruckes (IPAP) auf ein EPAP oder PEEP-Niveau welche umfasst; -eine regelbare Atemgasquelle (21), um einem IPAP-Druck bereitzustellen
- Sensor-Mittel (22), um Druck- und/oder Fluss-Werte des Atemgases zu ermitteln
- Prozessor-Mittel (23) zum Verarbeiten der vom Sensor ermittelten Werte
- Speicher-Mittel (24) zum abrufbaren Vorhalten von Funktionen
- Eingabemittel (2, 8) um die Funktionen zumindest teilweise zu bestimmen
- Steuermittel (25) welche die Atemgasquelle (21), entsprechend der Funktionen steuern, wobei die Steuermittel ausgehend vom IPAP-Druck in einer ersten Phase die Atemgasquelle zu einem Druckabfall einer ersten Charakteristik veranlassen und dann in einer zweiten Phase die Atemgasquelle zu einem zweiten Druckabfall mit einer von der ersten Phase abweichenden Charakteristik veranlassen.

Der Mikroprozessor (23) zur Verarbeitung der Sensorsignale (22) und/oder zur Steuerung des Motorlüfters (21) arbeitet vorzugsweise mit einer Taktfrequenz von mindestens 100 Hz, um auch für kurze Atemzüge während der Ausatmung in ausreichender Häufigkeit den Druck gemäß der jeweiligen Phase komfortabel zu regeln.

## Patentansprüche

1. Steuereinrichtung (20) für das Absenken eines Einatmungsdruckes (IPAP) auf ein EPAP oder PEEP-Niveau die Folgendes umfasst;
- eine regelbare Atemgasquelle (21), um einem IPAP-Druck bereitzustellen
- Sensor-Mittel (22), um Druck- und/oder Fluss-Werte des Atemgases zu ermitteln
- Prozessor-Mittel (23) zum Verarbeiten der vom Sensor ermittelten Werte
- Speicher-Mittel (24) zum abrufbaren Vorhalten von Funktionen
- Eingabemittel (2, 8) um die Funktionen zumindest teilweise zu bestimmen
- Steuermittel (25) welche die Atemgasquelle (21), entsprechend der Funktionen steuern, wobei die Steuermittel derart ausgestaltet sind, dass sie ausgehend vom IPAP-Druck in einer ersten Phase die Atemgasquelle zu einem Druckabfall einer ersten Charakteristik (14) veranlassen, mit einem Druckabfall im Bereich 30 - 300 hPa/sec und dann in einer zweiten Phase die Atemgasquelle zu einem zweiten Druckabfall (15, 16) mit einer von der ersten Phase abweichenden Charakteristik veranlassen, wobei der zweite Druckabfall wenigstens abschnittsweise im Bereich kleiner 30 hPa/sec liegt, **dadurch gekennzeichnet, dass** nach Beendigung der zweiten Funktion eine dritte Funktion angewendet wird, welche einen schnelleren Druckabfall steuert als die zweite Funktion.

2. Steuereinrichtung nach Anspruch 1 **dadurch gekennzeichnet, dass** die erste Phase eine Charakteristik mit einem Druckabfall im Bereich 50 - 20,0 hPa/sec aufweist.

3. Steuereinrichtung nach Anspruch 1 oder 2 **dadurch gekennzeichnet, dass** die erste Phase (I) eine Zeitdauer im Bereich 1% bis 10% der Ausatemzeit (Te) einnimmt.

4. Steuereinrichtung nach Anspruch 1, 2 oder 3 **dadurch gekennzeichnet, dass** der IPAP-Druck in der ersten Phase (I) um zumindest 25% abgesenkt wird.

5. Steuereinrichtung nach zumindest einem der vorhergehenden Ansprüche **dadurch gekennzeichnet, dass** die zweite Phase zumindest abschnittsweise eine Charakteristik mit konstantem Druck aufweist.

6. Steuereinrichtung nach zumindest einem der vorhergehenden Ansprüche **dadurch gekennzeichnet, dass** die zweite Phase (II) wenigstens abschnittsweise eine Charakteristik mit einem Druckabfall kleiner oder gleich 20 hPa/sec aufweist.

7. Steuereinrichtung nach zumindest einem der vorhergehenden Ansprüche **dadurch gekennzeichnet, dass** die zweite Phase (II) zwei verschiedene Charakteristiken (15, 16) mit unterschiedlichem Druckabfall aufweist.

8. Steuereinrichtung nach zumindest einem der vorhergehenden Ansprüche **dadurch gekennzeichnet, dass** die zweite Phase (II) zumindest drei verschiedene Charakteristiken mit unterschiedlichem Druckabfall aufweist.

9. Steuereinrichtung nach zumindest einem der vorhergehenden Ansprüche **dadurch gekennzeichnet, dass** eine der Charakteristiken der Phase II einen Steigung zwischen 30 und 300 hPa/sec aufweist.

10. Steuereinrichtung nach zumindest einem der vorhergehenden Ansprüche **dadurch gekennzeichnet, dass** die Übergänge zwischen den Charakteristiken eine diskontinuierliche Änderung der Steigung aufweisen.

11. Steuereinrichtung nach zumindest einem der vorhergehenden Ansprüche **dadurch gekennzeichnet, dass** die zweite Phase (II) wenigstens abschnittsweise eine Charakteristik mit sich stetig ändernder Steigung aufweist.

12. Steuereinrichtung nach zumindest einem der vorhergehenden Ansprüche **dadurch gekennzeichnet, dass** die zweite Phase (II) vor der nachfolgenden Umschaltung auf den Einatmungsdruck einen Abschnitt aufweist, in dem der Druck gleich dem endexspiratorischen Druck (PEEP) ist.

13. Steuereinrichtung nach zumindest einem der vorhergehenden Ansprüche **dadurch gekennzeichnet, dass** die zweite Phase erst zum Ende der Exspirationszeit den endexspiratorischen Druck (PEEP) erreicht.

14. Steuereinrichtung nach zumindest einem der vorhergehenden Ansprüche **dadurch gekennzeichnet, dass** über das Eingabemittel die Zeitdauer einer Phase und/oder der Druckabfall in einer Phase veränderbar ist.

## Claims

1. Control device (20) for lowering the inspiratory positive air pressure (IPAP) to an EPAP or PEEP level, which comprises the following:
a controllable breathing gas source (21) in order to provide an IPAP pressure
- sensor means (22) in order to determine the pressure and/or flow values of the breathing gas
- processor means (23) to process the values determined by the sensor
- storage means (24) for retrievable provision of functions
- input means (2, 8) in order to at least partially define the functions
- control means (25) which control the breathing gas source (21) according to the functions, wherein the control means is configured such that same on the basis of the IPAP pressure in a first phase cause a pressure drop of a first characteristic (14) in the breathing gas source, with a pressure drop in the region of 30 - 300 hPa/sec, and then in a second phase cause a second pressure drop (15, 16) in the breathing gas source, said second pressure drop comprising a characteristic which deviates from the first phase, wherein the second pressure drop lies at least in some sections in the region smaller than 30 hPa/sec, **characterised in that** when the second function has finished, a third function is applied which controls a pressure drop that is faster than the second function.

2. Control device according to claim 1, **characterised in that** the first phase comprises a characteristic which has a pressure drop in the region of 50 - 200 hPa/sec.

3. Control device according to claim 1 or 2, **characterised in that** the first phase (I) assumes a duration in the region of 1% to 10% of the exhalation time (Te).

4. Control device according to claim 1, 2 or 3, **characterised in that** the IPAP pressure in the first phase (I) is lowered by at least 25%.

5. Control device according to at least one of the preceding claims, **characterised in that** the second phase comprises at least in some sections a characteristic which has a constant pressure.

6. Control device according to at least one of the preceding claims, **characterised in that** the second phase (II) comprises at least in some sections a characteristic which has a pressure drop of 20 hPa/sec or lower.

7. Control device according to at least one of the preceding claims, **characterised in that** the second phase (II) comprises two different characteristics (15, 16) which have different pressure drops.

8. Control device according to at least one of the preceding claims, **characterised in that** the second phase (II) comprises at least three different characteristics which have different pressure drops.

9. Control device according to at least one of the preceding claims, **characterised in that** one of the characteristics of phase II comprises a second gradient between 30 and 300 hPa/sec.

10. Control device according to at least one of the preceding claims, **characterised in that** the transitions between the characteristics comprise a discontinuous change in gradient.

11. Control device according to at least one of the preceding claims, .**characterised in that** the second phase (II) comprises at least in some sections a characteristic which has a constantly changing gradient.

12. Control device according to at least one of the preceding claims, **characterised in that** the second phase (II) before the subsequent switching to the inspiratory pressure comprises a section in which the pressure is equal to the positive end-expiratory pressure (PEEP).

13. Control device according to at least one of the preceding claims, **characterised in that** the second phase only at the end of the expiratory time reaches the positive end-expiratory pressure (PEEP).

14. Control device according to at least one of the preceding claims, **characterised in that** the duration of a phase and/or the pressure drop in a phase can be changed via the input means.

## Revendications

1. Dispositif de commande (20) pour la réduction d'une pression inspiratoire (IPAP) pour atteindre un niveau EPAP ou PEP et qui comprend les éléments suivants :
- une source gaz respiratoire réglable (21) afin de fournir une pression IPAP
- un dispositif capteur (22) afin de déterminer une pression et/ou un débit de gaz respiratoire
- un dispositif processeur (23) afin de traiter les valeurs mesurées par le capteur
- un dispositif de stockage (24) afin de fournir des fonctions exécutables
- des dispositifs de saisie (2, 8) afin de définir au moins partiellement les fonctions
- des dispositifs de commande(25) pour piloter la source de gaz respiratoire (21) selon les fonctions, ces dispositifs de commande étant conçus de manière à permettre une réduction de la pression de la source de gaz respiratoire d'une première caractéristique (14) à partir de la pression IPAP, avec une réduction de la pression dans une plage située entre 30 et 300 hPa/sec, et ensuite dans une seconde phase (15, 16), de permettre une réduction de la pression de la source de gaz respiratoire d'une caractéristique différente de la première, la seconde réduction de pression étant au moins partiellement inférieure à 30 hPa/sec, **caractérisé en ce qu'**à la fin de la deuxième fonction une troisième fonction est exécutée, laquelle pilote une réduction de pression plus rapide que la deuxième fonction.

2. Dispositif de commande selon la revendication 1 **caractérisé en ce que** la première phase présente une caractéristique avec une diminution de pression située dans la page 50 - 200 hPa/sec.

3. Dispositif de commande selon la revendication 1 ou 2 **caractérisé en ce que** la première phase (I) présente une durée située dans la plage 1 % à 10 % de la durée expiratoire (Te).

4. Dispositif de commande selon la revendication 1, 2 ou 3 **caractérisé en ce que** la pression IPAP est réduite d'au moins 25 % au cours de la première phase (I).

5. Dispositif de commande selon au moins une des revendications précédentes **caractérisé en ce que** la deuxième phase présente au moins partiellement une caractéristique avec une pression constante.

6. Dispositif de commande selon au moins une des revendications précédentes **caractérisé en ce que** la deuxième phase (II) présente au moins partiellement une caractéristique avec une diminution de pression inférieure ou égale à 20 hPa/sec.

7. Dispositif de commande selon au moins une des revendications précédentes **caractérisé en ce que** la deuxième phase (II) présente deux caractéristiques différentes (15, 16) avec une diminution différente de la pression.

8. Dispositif de commande selon au moins une des revendications précédentes **caractérisé en ce que** la deuxième phase (II) présente au moins trois caractéristiques différentes avec une diminution différente de la pression.

9. Dispositif de commande selon au moins une des revendications précédentes **caractérisé en ce que** l'une des caractéristiques de la phase II présente une augmentation située entre 30 et 300 hPa/sec.

10. Dispositif de commande selon au moins une des revendications précédentes **caractérisé en ce que** la transition entre les caractéristiques présente une modification discontinue de l'augmentation.

11. Dispositif de commande selon au moins une des revendications précédentes **caractérisé en ce que** la deuxième phase (II) présente au moins partiellement une caractéristique avec une augmentation se modifiant constamment.

12. Dispositif de commande selon au moins une des revendications précédentes **caractérisé en ce que** la deuxième phase (II) présente, avant la commutation suivante sur la pression inspiratoire, une section dans laquelle la pression est égale à pression en fin d'expiration (PEP).

13. Dispositif de commande selon au moins une des revendications précédentes **caractérisé en ce que** la deuxième phase atteint la pression en fin d'expiration (PEP) seulement à la fin du temps expiratoire.

14. Dispositif de commande selon au moins une des revendications précédentes **caractérisé en ce que** la durée d'une phase et/ou la chute de pression est modifiable au cours d'une phase au moyen du dispositif de saisie.
